# EUROPEAN PATENT SPECIFICATION

(11) **EP 2 299 843 B1**
(45) Date of publication and mention of the grant of the patent: **21.02.2018**
(21) Application number: 08771181.8
(22) Date of filing: 16.06.2008
(51) Int. Cl.: A23K 20/105, A23K 20/147, A23K 40/10, A23K 50/48

(54) **COMPOSITION FOR ADDITION TO DRINKING WATER**
VERFAHREN FÜR DIE ANREICHERUNG VON TRINKWASSER
COMPOSITION POUR AJOUTER À L'EAU POTABLE

(43) Date of publication of application: 30.03.2011
(73) Proprietor: Hill's Pet Nutrition, Inc., Topeka, KS 66603 (US)
(72) Inventor: MARTINEZ, Sarah, Lawrence, KS 66047 (US); NADEAU, Douglas, Topeka, KS 66617 (US); CHINACHOTI, Pavinee, Topeka, KS 66615 (US); TAYLOR, Shawn, Silver Lake, KS 66539 (US)
(74) Representative: Wichmann, Hendrik
(86) International application number: PCT/US2008/067110
(87) International publication number: WO 2009/154608

(56) References cited:
- WO-A-2005/048714
- WO-A-2005/072759
- DE-A1- 3 427 014
- FR-A- 2 751 171
- FR-A- 2 773 554
- GB-A- 2 083 997
- US-A- 5 164 192
- US-A1- 2003 086 961
- DATABASE FSTA [Online] INTERNATIONAL FOOD INFORMATION SERVICE (IFIS), FRANkFURT-MAIN, DE; 1970, NISSHIN SANGYO CO: "Flavour enhancers." XP002514711 Database accession no. 71-2-03-t0104 & DATABASE WPI DERWENT PUBLICATIONS LTD., LONDON, GB; 1970-51945R 1970, NISSHIN SANGYO CO: "Increasing flavour and taste of seasonings" XP002514712

## Description

### FIELD OF THE INVENTION

The present invention relates to an effervescent composition for addition to the drinking water of an animal and a drink for use in hydration treatment of an animal.

### BACKGROUND OF THE INVENTION

Animal health, especially urinary health, is influenced not only by nutrition but also sufficient intake of water. Increased intake of water increases urine volume and dilutes mineral concentrations that are associated with lower urinary tract disease. Dogs and cats, for example, suffer from numerous disorders of the lower urinary tract. Among these are idiopathic urinary tract disease, crystalluria, bacterial cystitis, urolithiasis, idiopathic obstruction, urethral plugs and the like. Lower urinary tract disease (LUTD) is a disorder common to cats. Urolithiasis (stone formation in the urinary tract) is a condition commonly found in both dogs and cats. Although the etiology of these disorders is not completely clear, at least some of the factors associated with these disorders appear to be concentrated urine (*i.e.* high urine specific gravity) or high mineral supersaturation of urine. Lowering mineral concentrations in the urine by increasing urine production through increased water consumption can reduce the risk of urinary crystal or stone formation, assist in dissolving certain types of formed urinary tract stones, as well as reduce the occurrence of feline LUTD. In addition, increased urine volume initiates more frequent voiding. Frequent voiding reduces the risk of urinary tract infection, crystalluria and urolithiasis.

Whilst increasing an animal's water consumption is desirable, it is not readily achievable. Many animals, especially finicky animals like cats, older animals and sick animals may have no interest in increasing water intake. Moreover, where it is necessary to give the animal a micronutrient or functional ingredient as part of the drinking water, the animal may be aware that the water has an unusual taste and be unwilling to drink.

Palatants or flavourings may be added to animal drinking water. However, these are not always successful either in enticing the animal to drink or in masking the flavour of an added micronutrient or functional ingredient.

Effervescent technology has been used to provide compositions for both human and animal use. Effervescent technology is based on the reaction of an acid and a carbonate salt to form carbon dioxide and is known to provide self-dissolving compositions which may be added to water. For example, WO2005/072759 describes an effervescent composition which includes cranberry extract. Cranberries are often recommended for use in the treatment of urinary tract infections and an effervescent agent incorporating cranberry extract is described for human consumption. GB2083997 describes an animal feed supplement for use on pigs, poultry and calves, which comprises an effervescent composition for delivering micronutrients.

US5164192 discloses an effervescent composition for oral rehydration.

GB2083997 discloses an animal feed supplement.

XP002514711 (Japanese Patent 20549/70 (1970)) discloses a flavour enhancer containing fermentation gases.

FR2773554 discloses a supply of carbonated drinking water for domestic animals.

FR2751171 discloses an effervescent bait for fishing lines.

WO2005072759 discloses an effervescent composition including cranberry extract.

US20030086961 discloses an aqueous gel suitable for ingestion by cats or dogs comprising a gelling agent, a palatability enhancing agent, and water.

WO2005048714 discloses a highly palatable fluid composition for oral consumption which contains liver digest and results in increased total water intake and urine production and decreased urinary specific gravity in companion animals.

These examples of effervescent technology do not recognise or address the problem of enticing animals to increase water consumption.

Accordingly, a need exists for compositions effective in increasing water consumption in animals so as to reduce the risk of urinary tract disorders.

### BRIEF SUMMARY OF THE INVENTION

In a first aspect, the present invention provides an effervescent composition for addition to the drinking water of an animal, which composition comprises an effervescent agent and a palatant, wherein the palatant comprises a protein hydrolysate, wherein the effervescent agent comprises a combination of an acid and a carbonate base, wherein the weight ratio of acid:carbonate base is in the range of from 1:1 to 1:1.3, and wherein the composition is in the form of a tablet or powder.

In a still further aspect, the present invention provides a drink for use in the hydration treatment of an animal in which is dissolved an effervescent composition according to the invention.

The description further provides use of an effervescent composition comprising an effervescent agent and a palatant as an additive for animal drinking water.

The description further provides use of an effervescent composition comprising an effervescent agent and a palatant as a carrier for a micronutrient or functional ingredient for addition to animal drinking water.

The description further provides a method for treating an animal in need of hydration, which comprises adding an effervescent composition comprising an effervescent agent and a palatant to drinking water for the animal whereby the animal is encouraged to drink the drinking water.

The description further provides a method for encouraging an animal to drink, which comprises adding an effervescent composition comprising an effervescent agent and a palatant to drinking water for the animal.

The description further provides a method for delivering a micronutrient or functional ingredient to animal drinking water, comprising adding to the drinking water an effervescent composition comprising an effervescent agent, a palatant and the micronutrient or functional ingredient.

The description further provides a method for enhancing the taste of a palatant in animal drinking water, which comprises mixing an effervescent agent with the palatant and the drinking water, so as to encourage the animal to drink the water.

### BRIEF DESCRIPTION OF THE DRAWINGS

The present invention will now be described in further detail, by way of example only, with reference to the following examples and accompanying drawings, in which:
FIGURE 1 shows a comparison of cat fluid intake between conventional drinking water containing a palatant and water treated according to the present invention; and
FIGURE 2 shows a comparison of dog fluid intake between conventional drinking water containing a palatant and water treated according to the present invention.

### DETAILED DESCRIPTION OF THE INVENTION

It has surprisingly been found that a combination of the effervescent agent and palatant entices animals to drink more water than they would do so with a composition comprising the palatant alone. Without wishing to be bound by theory, it is thought that the effervescent agent enhances the taste of the palatant to the animal whereby the animal is encouraged to drink the water.

The invention is particularly applicable to non-human animals, such as mammals and is particularly applicable to companion animals, especially cats and dogs.

Non-human mammals include non-human primates such as monkeys, chimpanzees, *etc.* Farm animals include goats, sheep, swine, cattle, *etc.* Wild and zoo animals include wolves, bears, deer, giraffes, elephants, *etc.* Non-mammalian animals include birds. Working animals include horses.

Palatants as described herein are palatability enhancers which are typically employed to enhance the overall palatability of the drinking water and potentially to overcome any negative flavour effects arising from other components of the drinking water. Palatants are described in US2003/0086961. In the present invention, the palant comprises a protein hydrolysate. Particularly useful are protein hydrolysates which typically provide a savoury taste attractive to various animals including cats and dogs. The palatants include animal digest; animal hydrolysates; animal internal organs such as liver, lungs and heart; meats such as lamb, beef, pork, chicken and turkey; seafoods such as fish, crab, and shrimp; dairy products such as milk and cheese; yeasts; peptides; amino acids; nucleotides; fat; oil; artificial meat and/or seafood flavours; maillard reactants; sugars; plant extracts and other aromas nature and/or artificial that are attractive to the animals. For cats, preferred palatants include chicken liver hydrolysates and fish-based hydrolysates; for dogs, a protein digest/hydrolysate is preferred.

In the present invention, the effervescent agent comprises a combination of an acid and a carbonate base. Food grade acids may be used in the present invention, such as fruit acids. These acids include citric acid, malic acid, tartaric acid, adipic acid, and fumaric acid. Citric acid and malic acid are preferred because these are more soluble in water.

Typical bases used are carbonate salts such as sodium bicarbonate, potassium bicarbonate, sodium carbonate, potassium carbonate and mixtures thereof. The potassium salts may be used if there is a desire to keep sodium levels to a minimum in the effervescent composition.

The aim of the combination of the acid and base is to provide a reaction in water which generates carbon dioxide. This aids dissolution of the components of the effervescent composition and the reaction products may impart a salty flavour to the drinking water. It is also thought that application of the effervescent composition to the water may provide carbon dioxide bubbles which themselves deliver some palatant aroma from the drinking water which may attract the animals to the water.

In the present invention, the weight ratio of acid to salt is in the range of from 1:1 to 1:1.3. The exact ratio may be determined according to how quickly the composition needs to dissolve and exactly how much the taste of the composition is altered by the amount of acid or base present therein.

In addition to the effervescent agent, the composition typically comprises one or more additional components selected from flavourings, sweeteners, colours, micronutrients and functional ingredients. The flavourings, sweeteners and colours may be any conventional components suitable for this purpose. These components may enhance the attractiveness of the drinking water to the animal. Where the additional components include micronutrients and/or functional ingredients, the composition according to the invention may act as a delivery vehicle for these components. Micronutrients include vitamins and minerals and functional ingredients include pharmaceutically active components, antioxidants such as lipoic acid and plant extracts such as phenolics, proanthrocyanidin and green tea, as well as taurine specifically for cats, amino acids and probiotics (live microorganisms that help improve gut flora). According to the present invention, these additional components may readily be delivered using the effervescent composition which may mask any unpleasant taste associated with the micronutrients or functional ingredients. In addition, the effervescent composition of the present invention may be used to deliver heat labile functioning ingredients or nutrients. This is because the effervescent composition does not require heat to operate. Probiotics and phytochemicals such as green tea extracts and antioxidants are heat labile could not readily be delivered in a process requiring heating.

Cranberry juice extract is a further functional ingredient which may be delivered according to the present invention. Cranberry juice is often recommended for overall urinary tract health.

Advantageously, the micronutrient or functional ingredients may comprise soluble fibre. According to this arrangement, an effervescent composition made according to the invention incorporating soluble fibre may be used as an additive for an animal's drinking water to induce weight loss in an animal, particularly an overweight animal. Presence of the fibre in the water would typically give the animal a sensation of a full stomach thereby suppressing appetite.

The effervescent composition according to the invention is in the form of a tablet or powder. Tablet form is particularly useful because this may be conveniently packaged in a tube or in a foil or blister packet enabling convenient storage and portability by the user. Conventional ingredients may be added to the effervescent composition to provide a formulation suitable for use as a powder or for tableting. For example, binders are normally added to effervescent tablets. To ensure a suitable hardness the binder should preferably be water-soluble and may typically be selected from dextrose, lactose, sorbitol and xylitol. The amount of binder should make the tablet hard enough to be robust in handling but nevertheless soft enough to dissolve in the drinking water. A water-soluble lubricant may be added to the composition for certain types of tablet pressing and typical water soluble lubricants include polyethylene glycol, adipic acid and sodium benzoate. The composition may be tableted using standard tablet pressing with or without a granulation step prior to the tablet pressing. Granules for effervescent tablets may be manufactured by wet fusion or by heat fusion. In wet fusion, the acid component is moistened and added to the base. Using a suitable mixture, granules may be formed by kneading and are tableted whilst still damp. Further details of this process are to be found in "The Theory and Practice of Industrial Pharmacy (1970)" pages 326 to 327 (edited by Lachman *et al*). Drying ovens may be used to dry the product. Fluid bed dryers and vacuum granulators have also been used to make effervescent granulations.

It is important during the production of tablets or powders to ensure that humidity is strictly controlled throughout. The packaging used for the product must also control humidity. Tablets should be packaged with material of a high moisture barrier and may advantageously include a dessicant built into the package system.

The effervescent composition should advantageously be capable of disintegration in the drinking water within five minutes, preferably within three minutes, more preferably within two minutes or less.

### EXAMPLES

### Example 1

Five effervescent compositions were formulated, each comprising an effervescent agent comprising citric acid and sodium bicarbonate in a ratio 1:1.3. Each of the five separate compositions contained a different palatant A to E which was a conventional feline palatant comprising a chicken liver hydrolysate or a fish-based hydrolysate. Drinking water containing this composition was compared with drinking water containing the palatant alone in a test involving a population of twenty five cats. The cats were healthy adult male and female cats of at least one year of age. Each cat was individually caged in a cage containing a feeding bowl and two drinking bowls. The cats were fed on Hill's Pet Nutrition Science Diet Feline Adult Dry Cat Food using amounts in each bowl to maintain ±2% body weight according to the feeding guide. One of the drinking bowls contained 200g of tap water. The other drinking bowl contained the same amount of drinking water with 1 tablet of effervescent composition. Each bowl was weighed before placing in the cage and again after removing from the cage so as to measure intake. The effervescent solution, water and food bowls were removed at the same time daily. The test took place over 2 days.

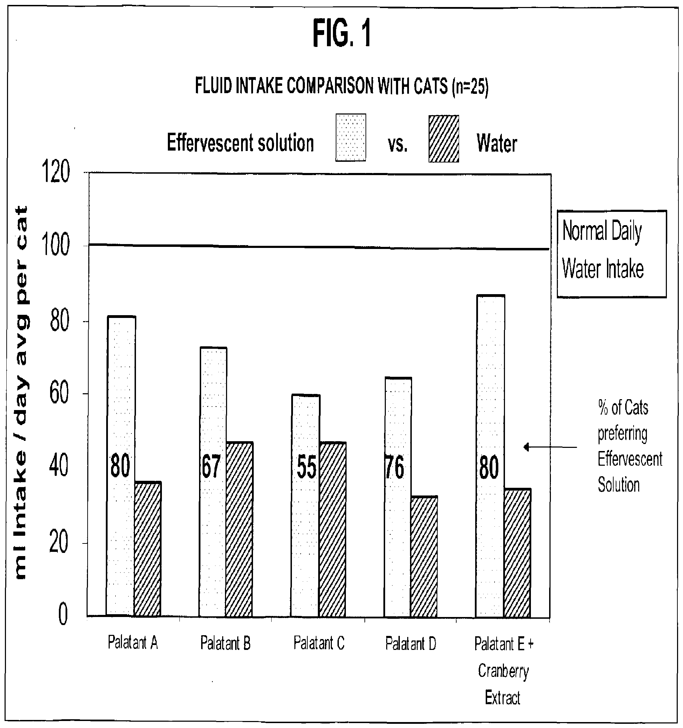

It will be apparent from Figure 1 that the drinking water containing the effervescent composition according to the invention was consumed in significantly greater quantity by the cats as compared with the drinking water containing the palatant alone. This indicates that the compositions according to the invention encourage cats to drink more drinking water.

### Example 2

An analogous test was performed in relation to dogs. A population of 10 dogs was subjected to the same 2 day test as for cats. The dogs were healthy adult male and female dogs of at least one year of age. For the dogs the food used was Hill's Pet Nutrition Science Diet Canine Adult Original Dry Dog Food and 500g tap water was used for drinking water. For the effervescent composition, 1 tablet was used in 500g of water.

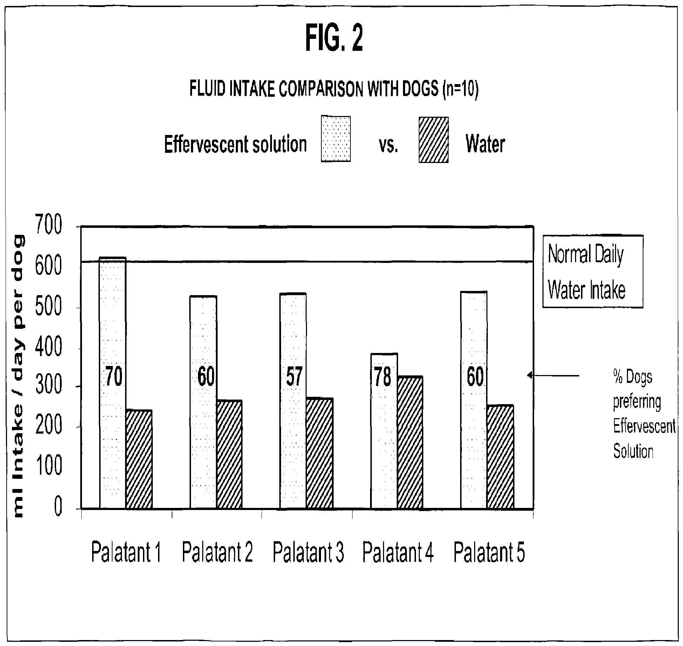

Again, five different effervescent compositions were compared, each with a different palatant (1-5) and the results are shown in Figure 2 for a population of ten dogs. Again, it was found that dogs were encouraged to drink more water when presented with the drinking water containing the effervescent composition of the present invention.

## Claims

1. An effervescent composition for addition to the drinking water of an animal, which composition comprises an effervescent agent and a palatant, wherein the palatant comprises a protein hydrolysate,
wherein the effervescent agent comprises a combination of an acid and a carbonate base,
wherein the weight ratio of acid:carbonate base is in the range of from 1:1 to 1:1.3, and
wherein the composition is in the form of a tablet or powder.

2. A composition according to claim 1, wherein the acid is selected from citric acid, malic acid, tartaric acid, adipic acid, fumaric acid, and mixtures thereof.

3. A composition according to claim 1 or claim 2, wherein the carbonate base is a salt selected from sodium bicarbonate, potassium bicarbonate, sodium carbonate, potassium carbonate and mixtures thereof.

4. A composition according to any preceding claim, which further comprises one or more additional components selected from flavourings, sweeteners, colours, micronutrients, and functional ingredients.

5. A composition according to claim 4, which further comprises cranberry juice.

6. The composition according to claim 4, wherein the functional ingredients comprise soluble fibre.

7. The composition according to claim 4, wherein the functional ingredients comprise a heat-labile functional ingredient selected from probiotics and phytochemicals.

8. The composition according to any preceding claim, for use in the hydration treatment of an animal.

9. The composition according to claim 6, for use in inducing weight loss in an overweight animal.

10. A drink for use in hydration treatment of an animal, in which is dissolved an effervescent composition according to any of claims 1 to 7.

11. Use of a composition according to any one of claims 1 to 7 as an additive for animal drinking water.

12. Use of a composition according to any one of claims 1 to 7 as a carrier for a micronutrient or functional ingredient for addition to animal drinking water.

## Patentansprüche

1. Brausezusammensetzung zur Zugabe zum Trinkwasser eines Tieres, welche Zusammensetzung ein Brausemittel und einen Geschmacksträger umfasst, wobei der Geschmacksträger ein Proteinhydrolysat umfasst,
wobei das Brausemittel eine Kombination aus einer Säure und einer Carbonatbase umfasst,
wobei das Gewichtsverhältnis von Säure:Carbonatbase im Bereich von 1:1 zu 1:1,3 liegt, und
wobei die Zusammensetzung in der Form einer Tablette oder eines Pulvers ist.

2. Zusammensetzung nach Anspruch 1, wobei die Säure aus Zitronensäure, Äpfelsäure, Weinsäure, Adipinsäure, Fumarsäure und Gemischen davon ausgewählt ist.

3. Zusammensetzung nach Anspruch 1 oder Anspruch 2, wobei die Carbonatbase ein Salz ist, das aus Natriumbicarbonat, Kaliumbicarbonat, Natriumcarbonat, Kaliumcarbonat und Gemischen davon ausgewählt ist.

4. Zusammensetzung nach einem beliebigen voranstehenden Anspruch, die weiterhin einen oder mehrere zusätzliche Bestandteile umfasst, der/die aus Geschmacksstoffen, Süßungsmitteln, Farbstoffen, Mikronährstoffen und funktionellen Bestandteilen ausgewählt ist/sind.

5. Zusammensetzung nach Anspruch 4, die weiterhin Kranbeersaft umfasst.

6. Zusammensetzung nach Anspruch 4, wobei die funktionellen Bestandteile lösliche Faser umfassen.

7. Zusammensetzung nach Anspruch 4, wobei die funktionellen Bestandteile einen hitzelabilen funktionellen Bestandteil umfassen, der aus Probiotika und Phytochemikalien ausgewählt ist.

8. Zusammensetzung nach einem beliebigen voranstehenden Anspruch zur Verwendung in der Flüssigkeitszufuhrbehandlung eines Tieres.

9. Zusammensetzung nach Anspruch 6 zur Verwendung beim Induzieren von Gewichtsabnahme in einem übergewichtigen Tier.

10. Getränk zur Verwendung bei der Flüssigkeitszufuhrbehandlung eines Tieres, in dem eine Brausezusammensetzung nach einem beliebigen der Ansprüche 1 bis 7 gelöst ist.

11. Verwendung einer Zusammensetzung nach einem beliebigen der Ansprüche 1 bis 7 als einen Zusatz zu Tiertrinkwasser.

12. Verwendung einer Zusammensetzung nach einem beliebigen der Ansprüche 1 bis 7 als einen Träger für einen Mikronährstoff oder einen funktionellen Bestandteil zur Zugabe zu Tiertrinkwasser.

## Revendications

1. Composition effervescente destinée à être ajoutée à l'eau de boisson d'un animal, laquelle composition comprend un agent effervescent et un agent de sapidité, dans laquelle l'agent de sapidité comprend un hydrolysat de protéines,
dans laquelle l'agent effervescent comprend une combinaison d'un acide et d'une base carbonate,
dans laquelle le rapport pondéral de l'acide:base carbonate est compris entre 1:1 et 1:1,3, et
dans laquelle la composition est sous la forme d'un comprimé ou d'une poudre.

2. Composition selon la revendication 1, dans laquelle l'acide est choisi parmi l'acide citrique, l'acide malique, l'acide tartrique, l'acide adipique, l'acide fumarique et les mélanges de ceux-ci.

3. Composition selon la revendication 1 ou la revendication 2, dans laquelle la base carbonate est un sel choisi parmi le bicarbonate de sodium, le bicarbonate de potassium, le carbonate de sodium, le carbonate de potassium et les mélanges de ceux-ci.

4. Composition selon l'une quelconque des revendications précédentes, qui comprend en outre un ou plusieurs constituants supplémentaires choisis parmi les arômes, les édulcorants, les colorants, les micronutriments et les ingrédients fonctionnels.

5. Composition selon la revendication 4, qui comprend en outre du jus de canneberge.

6. Composition selon la revendication 4, dans laquelle les ingrédients fonctionnels comprennent une fibre soluble.

7. Composition selon la revendication 4, dans laquelle les ingrédients fonctionnels comprennent un ingrédient fonctionnel thermolabile choisi parmi les probiotiques et les composés phytochimiques.

8. Composition selon l'une quelconque des revendications précédentes, pour une utilisation dans le traitement d'hydratation d'un animal.

9. Composition selon la revendication 6, pour une utilisation dans l'induction de la perte de poids chez un animal en surpoids.

10. Boisson pour une utilisation dans un traitement d'hydratation d'un animal, dans laquelle est dissoute une composition effervescente selon l'une quelconque des revendications 1 à 7.

11. Utilisation d'une composition selon l'une quelconque des revendications 1 à 7 en tant qu'additif pour l'eau de boisson d'un animal.

12. Utilisation d'une composition selon l'une quelconque des revendications 1 à 7 en tant que support pour un micronutriment ou un ingrédient fonctionnel destinée à être ajoutée à l'eau de boisson d'un animal.
